# EUROPEAN PATENT SPECIFICATION

(11) **EP 2 440 148 B1**
(45) Date of publication and mention of the grant of the patent: **20.07.2016**
(21) Application number: 10786817.6
(22) Date of filing: 09.06.2010
(51) Int. Cl.: A61B 17/88, A61B 17/70, A61B 17/80

(54) **LAMINOPLASTY SYSTEM**
LAMINOPLASTIESYSTEM
SYSTÈME DE LAMINOPLASTIE

(30) Priority: 09.06.2009 US 185360 P
(43) Date of publication of application: 18.04.2012
(73) Proprietor: Robinson, James, C, Atlanta, GA 30339 (US)
(72) Inventor: Robinson, James, C, Atlanta, GA 30339 (US)
(74) Representative: Leissler-Gerstl, Gabriele
(86) International application number: PCT/US2010/038056
(87) International publication number: WO 2010/144636

(56) References cited:
- JP-A- 2008 534 119
- KR-B1- 100 897 928
- US-A1- 2003 125 740
- US-A1- 2004 030 388
- US-A1- 2004 030 388
- US-A1- 2005 273 100
- US-A1- 2008 140 129
- US-B1- 6 491 695

## Description

### Field of the Invention

The present invention relates generally to a surgical system, most particularly for use in performing a laminoplasty to treat cervical stenosis in the spine. More specifically, the invention relates to a system for increasing the area of the spinal canal by securing a separated lamina portion of a desired cervical vertebra in a relief position.

### Background of the Invention

Spinal stenosis is a pathology of the spine that involves the narrowing of the spinal canal, through which the spinal cord and nerve roots run. This narrowing may be congenital and, consequently, can affect patients at any age. Spinal stenosis may result from thickening and calcification of spinal ligaments. For example, calcification can result from deposits of calcium salts within the spine. In addition, spinal stenosis can result when bones and joints are enlarged, leading to the formation of osteophytes (bone spurs). A significant cause of osteophytes is spondylosis, in which spinal discs lose water and become less dense. Also, a herniated disc may place pressure on the spinal cord or nerve root such that the area of the spinal canal is reduced. Finally, diseased bone or tumors can extend into the spinal cord area, decreasing the space available for nerve roots within the spinal canal.

Compression of the spinal cord resulting from spinal stenosis can produce pain, weakness, or loss of feeling in the patient. Additionally, spinal cord compression can lead to myelopathy, which causes neurological damage and results in spinal cord malfunction. If left untreated, the compression can eventually damage the circulatory system within the spinal cord, leading to more severe myelopathy.

Two surgical methods are traditionally used to decompress the spinal cord. First, the laminectomy involves the removal of the lamina and spinous processes in order to expose the dura covering the spinal cord. Due to the removal of portions of the supporting structures at the posterior of the vertebra that are used to align the spinal column, a laminectomy can create postural deformities in patients. In addition, there is a risk that the procedure will lead to substantial scar formation in the patient. In order to address these concerns, a graft may be installed between the vertebral bones involved to promote fusion. However, this may lead to a decrease in the range of motion in the spine, and there may also be accelerated degeneration of the vertebrae above and below the repaired vertebra.

The second method traditionally used to decompress the spinal cord is the laminoplasty. In a laminoplasty procedure, the targeted vertebra is cut and spread apart so that the lamina is lifted off the dura and the spinal canal is thus enlarged. Then, a plate and/or a graft are inserted to permanently enlarge the spinal canal. There are generally two techniques used to perform a laminoplasty. First, the unilateral or "open door" laminoplasty involves cutting entirely through a first portion of the lamina on the first side of midline of the targeted vertebra, while a second portion of the lamina on the second side of midline is only cut partially through to create a hinge. Then, the first lamina portion is hinged away from the spinal cord to increase the size of the spinal canal. Finally, a graft and/or plate is inserted into the opening to permanently enlarge the spinal canal. Second, the bilateral or "French door" laminoplasty involves cutting entirely through the midline of the spinous process, and then cutting partially through both sides of the lamina portion, creating two hinges. The vertebra can then be opened at the bisected spinous process, and a graft or plate can be inserted into the opening to permanently enlarge the spinal canal.

Unlike the laminectomy, the laminoplasty does not involve the excising of any bone material. In addition, when compared to the laminectomy, the laminoplasty provides greater stability. A wider range of motion for the patient is maintained compared to a fusion. Through the use of laminar fusion and fixation techniques in a laminoplasty procedure, the achieved decompression and position of the displaced lamina can be more effectively maintained.

Despite the advances that have been achieved in laminoplasty procedures, there are still some limitations in the effectiveness of the procedures and the ease with which the procedures are completed, especially when performed on the cervical vertebrae. For example, the present technique requires the surgeon to make a large incision to reach the spine, which includes stripping of muscle and ligament attachments to the bone, and this can lead to significant muscle and tissue damage. In addition, in cervical spine surgeries, the smaller size of the target vertebra makes the operation more complicated. For instance, the surgeon may find it difficult to make precise adjustments within the operating space or to know whether the lamina has been displaced an appropriate distance. Further, in some patients, the increase in area that can be achieved by current techniques is insufficient to provide complete relief from spinal cord compression. Finally, due to the uneven nature of "open door" laminaplasties, patients may have a slight imbalance in their spines following the procedure, and the increase in spinal canal diameter is asymmetric.

Similarly, the laminoplasty plates that are currently used also have limitations. For example, many current laminoplasty plates are too large in size for insertion into small incisions or for effective attachment to cervical vertebrae. In addition, current plates frequently lack the stability required to permanently orient the lamina in an appropriate position. Also, the design of existing laminoplasty plates often makes the process of attaching the plate to the vertebra and lamina very challenging. Finally, many existing laminoplasty plates are not adequately constructed to allow for conjunctive use of bone fusion material. Existing plates are also cumbersome for use with less invasive surgical procedures.

Examples of system for use in performing a laminoplasty to treat cervical stenosis in the spine can be found in US 2004/030388 A1, JP 2008 534119 A, US 2005/273100 A1, KR 100897928 B1. A system for performing a laminoplasty for treating cervical stenosis in a patient with the features as defined in the preamble of claim 1 is known from JP2008 534119 A.

Accordingly, it remains desirable in the pertinent art to provide laminoplasty plates to address the limitations associated with known plates, including but not limited to those limitations discussed above. Additionally, it is desirable in the pertinent art to provide methods and systems for using the said laminoplasty plates to address the limitations associated with known methods and systems, including but not limited to those limitations discussed above.

### SUMMARY

Presented herein is a laminoplasty plate as defined in the claims for securing a separated lamina portion of a desired cervical vertebra in a relief position and a method not claimed, of using the same to perform a laminoplasty. The laminoplasty plate comprises a proximal end portion having a bottom surface defined in a first plane and a distal end portion having a bottom surface defined in a second plane.

A lamina setting tool as defined in the claims is presented for positioning the lamina portion of the desired cervical vertebra in the relief position. The tool comprises a rotatable threaded shaft comprising an adjustable stop and a guide having a body portion and a first support arm. In this aspect, the body portion has a fixed length and is spaced therefrom the rotatable threaded shaft. The guide is coupled to the rotatable threaded shaft by the first support arm, which is connected to the body portion. At the distal end of the first support arm, the guide is coupled to the rotatable threaded shaft. In this aspect, the stop is selectively adjustable to correspond to a given limited depth, which will prevent over penetration of the threaded member through the underside of the lamina into the spinal canal.

The lamina portion can be controllably elevated to a relief position in which the spinal canal of the desired cervical vertebra has a relief cross-sectional area that is greater than the pre-operative cross-sectional area, wherein the lamina portion is subsequently secured in an elevated position.

The lamina setting tool can be provided to assist with the step of controllably raising and securing the lamina portion in the relief position. In this aspect, the guide is configured to detachably mount to the mountable portion of the laminoplasty plate.

Related methods, not claimed, of operation are also provided. Other apparatuses, methods, systems, features, and advantages of the laminoplasty plates and the method of their use will be or become apparent to one with skill in the art upon examination of the following figures and detailed description. It is intended that all such additional apparatuses, methods, not claimed, systems, features, and advantages be included within this description, be within the scope of the laminoplasty plates and the method, not claimed, of their use, and be protected by the accompanying claims.

### DESCRIPTION OF THE FIGURES

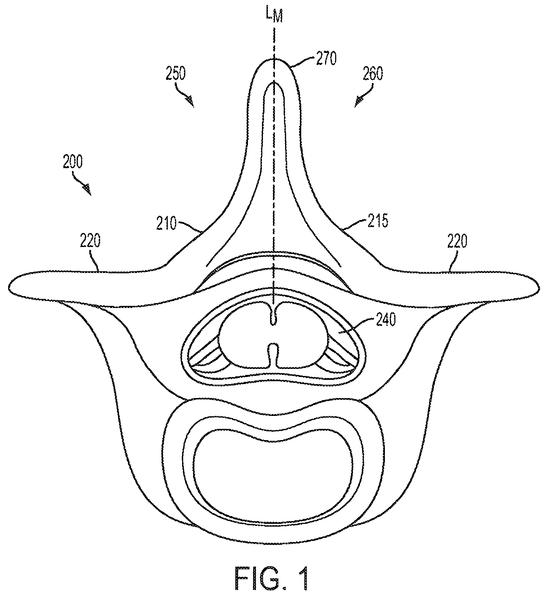

FIG. 1 is top plan view of an exemplified cervical vertebra showing the spinal canal having a pre-operative cross-sectional area.
FIG. 2 is a top plan view of the cervical vertebra of FIG. 1, showing a first lamina portion in the relief position.
FIG. 3 is a top plan view of the cervical vertebra of FIG. 1, showing the first and second lamina portions in the relief position.

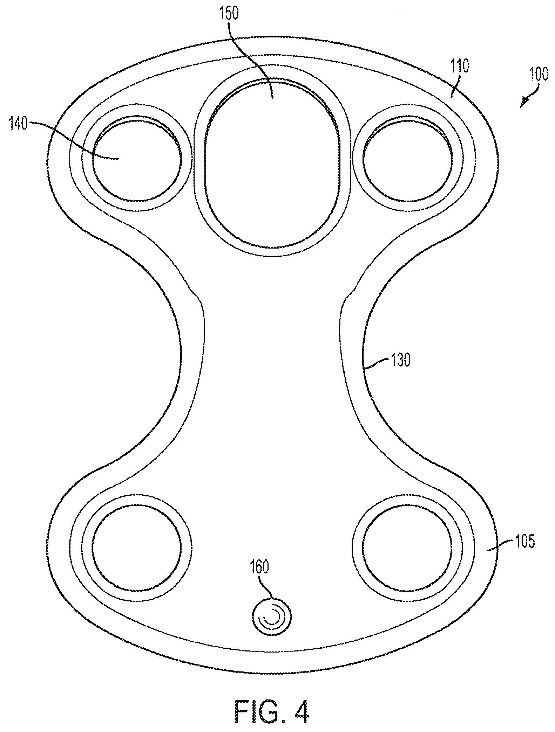

FIG. 4 is a top plan view of an exemplified laminoplasty plate.

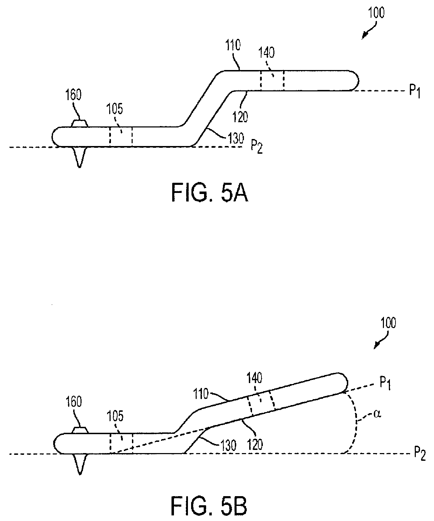

FIG. 5A is a side elevational view of the laminoplasty plate of FIG. 4, showing the first and second planes in parallel.
FIG. 5B is a side elevational view of the laminoplasty plate of FIG. 4, showing the first and second planes are at an acute angle relative to one another.

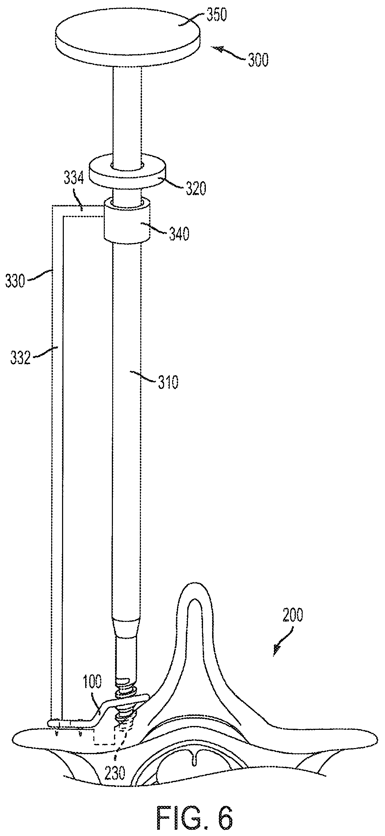

FIG. 6 is a side elevational view of a lamina setting tool. (for reference)
FIG. 7 is a partially exploded perspective view of a lamina setting tool.
FIG. 8 is a back side elevational view of the lamina setting tool of FIG. 7.
FIG. 9 is a front side elevational view of the lamina setting tool of FIG. 7.

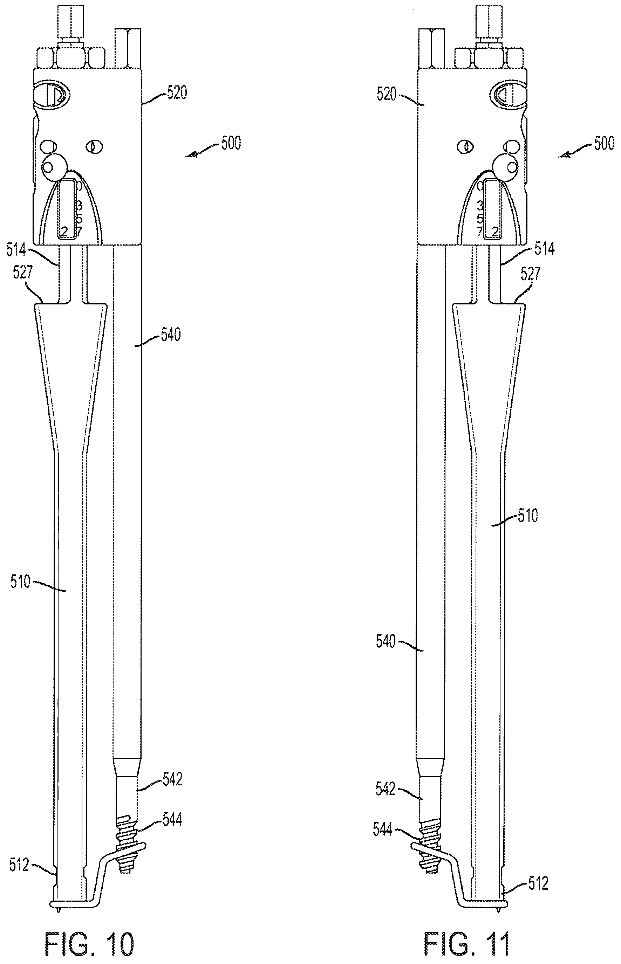

FIG. 10 is a right side elevational view of the lamina setting tool of FIG. 7.
FIG. 11 is a left side elevational view of the lamina setting tool of FIG. 7.

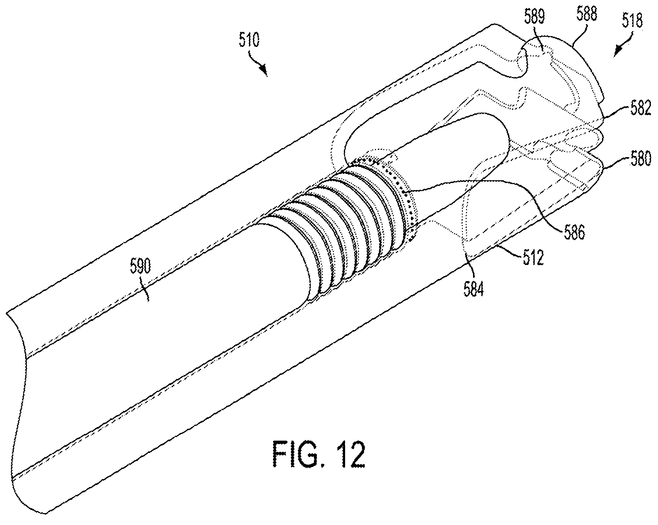

FIG. 12 is a partially transparent perspective view of the distal end of the elongated guide of the lamina setting tool of FIG. 7.
FIG. 13 is a cut away side elevational view of the distal end of the elongated guide of the lamina setting tool of FIG. 7.

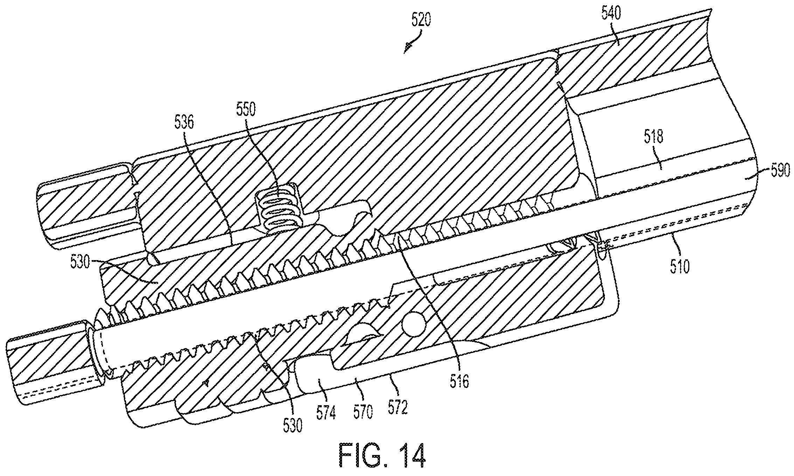

FIG. 14 is a cut away perspective view the housing of the lamina setting tool of FIG. 7.

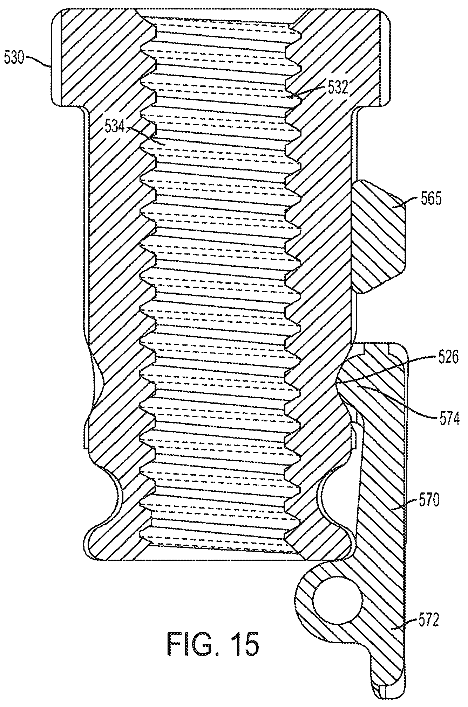

FIG. 15 is a cut away side elevational view of the drive nut of the lamina setting tool of FIG. 7.

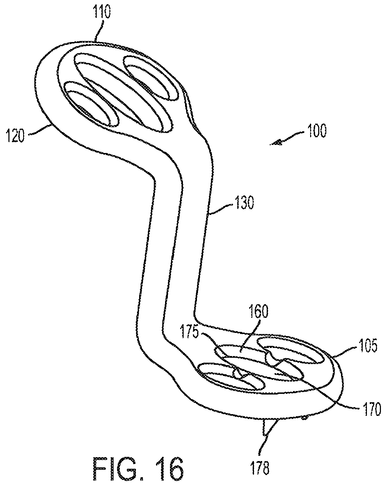

FIG. 16 is a perspective view of a laminoplasty plate, according to one aspect.

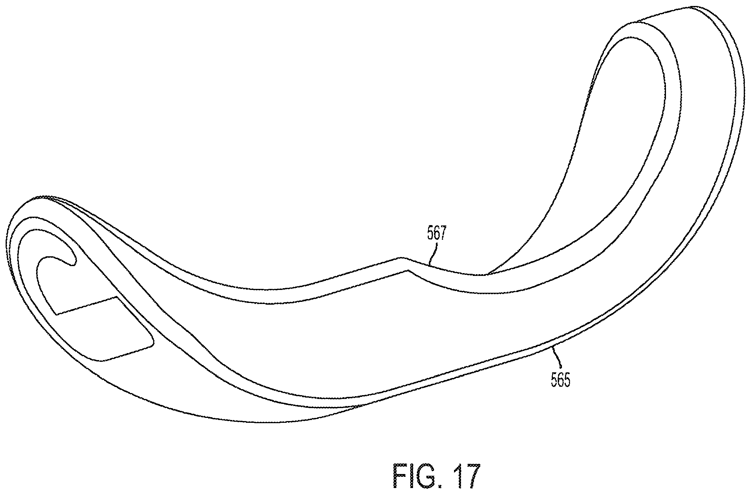

Fig. 17 is a perspective view of the lockslide of the lamina setting tool of Fig. 7.

### DESCRIPTION OF THE INVENTION

The present invention can be understood more readily by reference to the following detailed description, examples, and claims, and their previous and following description. Before the present system and/or devices, methods are disclosed and described, it is to be understood that this invention is not limited to the specific systems and/or devices disclosed unless otherwise specified, as such can, of course, vary. It is also to be understood that the terminology used herein is for the purpose of describing particular aspects only and is not intended to be limiting.

The following description of the invention is provided as an enabling teaching of the invention in its best, currently known aspect. Those skilled in the relevant art will recognize that many changes can be made to the aspects described, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention. Thus, the following description is provided as illustrative of the principles of the present invention and not in limitation thereof.

As used herein, the singular forms "a," "an" and "the" include plural referents unless the context clearly dictates otherwise. Thus, for example, reference to a "plate" includes aspects having two or more plates unless the context clearly indicates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

As used herein, the terms "optional" or "optionally" mean that the subsequently described event or circumstance may or may not occur, and that the description includes instances where said event or circumstance occurs and instances where it does not.

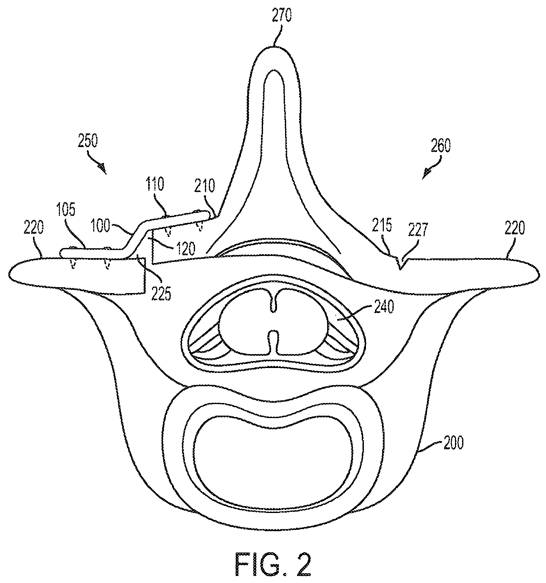

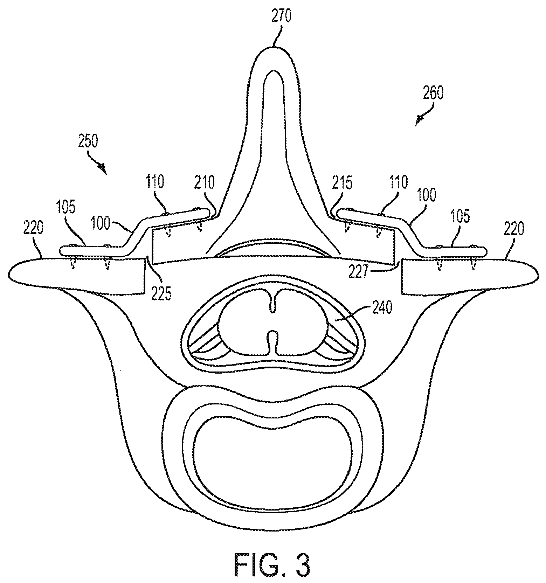

In one aspect, presented herein is a laminoplasty plate 100 for securing a separated lamina portion 210 of a desired cervical vertebra 200 in a relief position, as shown in Figs. 2 and 3. In one aspect, the laminoplasty plate 100 comprises a proximal end portion 110 having a bottom surface 120 defined in a first plane P₁ and a distal end portion 105 having a bottom surface 120 defined in a second plane P₂. In this aspect, the first and second planes are spaced from one another at the medial portion 130 such that the bottom surface of the proximal end portion 110 is spaced from the bottom surface of the distal end portion 105. In one exemplified aspect, the first plane is spaced a predetermined distance of between about 1 mm and about 10 mm. In another example, the first and second planes are spaced between about 3 mm and about 7mm. In one aspect, the laminoplasty plate can be comprised of biocompatible materials such as, and not meant to be limiting, titanium, titanium alloys, surgical steel, polymeric material, ceramic material, carbon fiber composite, resorbable material, polyglyconate, autograft bone, allograft bone, xenograft bone, and hydroxy-apatite.

In one exemplified aspect, the first plane P₁ can be substantially parallel to the second plane P₂. Alternatively, the first plane can be at an acute angle α relative to the second plane. Preferably, the acute angle is between about 0 degrees and 89 degrees, and more preferably between about 0 degrees and 30 degrees. In another aspect, the laminoplasty plate can comprise a medial portion 130 that is connected to the proximal end portion and the distal end portion. In one exemplified aspect, the medial portion 130 can be arcuate in shape. In another example, the medial portion can have a reduced cross-sectional area relative to the cross-sectional areas of the distal and proximal end portions. As one skilled in the art will appreciate, this reduced cross-sectional area permits the medial portion to be more fully surrounded by bone fusion material. In a further example, the medial portion can be comprised of a substantially rigid material and have an increased cross-sectional area relative to the cross-sectional areas of the distal and proximal end portions. As one skilled in the art will appreciate, this rigidity and increase in cross-sectional area make the laminoplasty plate 100 more resistant to tensile, compressive, or shear loads while allowing the distal and proximal end portions to remain substantially flat.

In one aspect, the laminoplasty plate defines a plurality of bores 140 of pre-determined diameters. In one exemplified aspect, the proximal end portion 110 defines two paired and opposing screw bores 140 that extend substantially transverse therethrough the proximal end portion between the top and bottom surfaces of the proximal end portion and that are configured to operatively receive screws. In another aspect, the proximal end portion also defines a tool screw aperture 150 that extends substantially transverse therethrough the proximal end portion between the top and bottom surfaces of the proximal end portion and is configured to operatively receive a portion of a lamina setting tool 300. In another aspect, the tool screw aperture can be positioned therebetween the pair of screw bores of the proximal end portion. In this aspect, it is contemplated that the tool screw aperture can be positioned adjacent to and equidistant from each screw bore. In a further aspect, the distal end portion defines two paired opposing bores that extend substantially transverse therethrough the distal end portion between the top and bottom surfaces of the distal end portion and is configured to operatively receive screws. It is contemplated that different quantities and positions of the bores of the laminoplasty plate can be used in the present invention.

In one aspect, the distal end portion is comprised of a mountable portion 160 configured for detachably mounting a guide 330. In one exemplified aspect, the mountable portion 160 can be comprised of a raised cone portion to which the guide 330 can be mounted. In another example, the mountable portion can define a cavity to which the guide can be mounted. In another aspect, the mountable portion can define a tool bore 170 for receipt of a portion of the lamina setting tool. In another aspect, the mountable portion can be positioned therebetween the pair of screw bores 140 of the distal end portion 105. In this aspect, it is contemplated that the mountable portion can be positioned adjacent to and equidistant from each screw bore. Further, it is contemplated that different positions and configurations of the mountable portion of the laminoplasty plate 100 can be used in the present invention.

For reference, a lamina setting tool 300 is presented for positioning the lamina portion 210 of the desired cervical vertebra 200 in the relief position. The tool comprises a rotatable threaded shaft 310 comprising an adjustable stop 320 and a guide having a body portion 332 and a first support arm 334. The body portion 332 has a fixed length and is spaced therefrom the rotatable threaded shaft. The guide is coupled to the rotatable threaded shaft 310 by the first support arm 334, which is connected to the body portion. At the distal end of the first support arm, the guide is coupled to the rotatable threaded shaft. Further, the body portion of the guide is secured to a stable structure. In one example, the body portion is secured to the desired cervical vertebra substantially on or near the lateral mass 220. In another example, the body portion is secured to a stable structure located within the operating room, such as the operating room table. The rotatable threaded shaft and the body portion of the guide 330 are substantially parallel to one another. The lamina setting tool can be comprised of biocompatible materials such as, and not meant to be limiting, titanium, titanium alloys, surgical steel, polymeric material, ceramic material, carbon fiber composite, resorbable material, polyglyconate, autograft bone, allograft bone, xenograft bone, and hydroxy-apatite.

The stop 320 is selectively adjustable to correspond to a given limited depth, which will prevent over penetration ofithe threaded member through the underside of the lamina into the spinal canal 240. The stop is adjustable along the length of the rotatable threaded shaft. The rotatable threaded shaft is attached to the lamina portion at an attachment point 230. In one example the guide can comprise a second support arm (not shown) that is coupled to the rotatable threaded shaft 310 at a position between the first support arm and the attachment point 230. There may also be a small cylinder 340 connected to the distal end portion of the first support arm. The threaded shaft is configured to fit through and be laterally supported by the small cylinder 340. In another example, the rotatable threaded shaft can comprise a handle portion 350. In this example, the handle portion 350 can be positioned at the top of the rotatable threaded shaft to allow for easy rotation of the rotatable threaded shaft. In a further example, the lamina setting tool comprises a gauge (not shown) calibrated to measure a lift distance. In this example, it is contemplated that the gauge can enable the user of the lamina setting tool to monitor the amount of rotation necessary to achieve a particular lift distance. Specifically, it is contemplated that the gauge can include a means for tracking the number of full rotations of the rotatable threaded shaft, where one full rotation of the rotatable threaded shaft corresponds to a particular lift distance. of the present invention

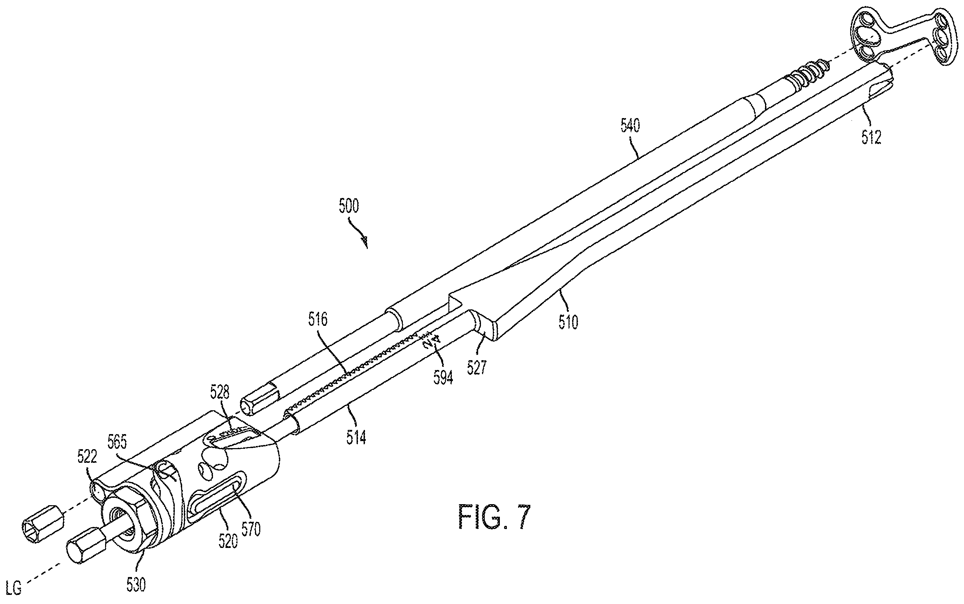

The lamina setting tool 500, of the present invention as shown in Fig. 7, comprises an elongate guide 510 with a the distal end 512 configured to engage portions of the laminoplasty plate 100 at or near its mountable portion. As illustrated, the lamina setting tool comprises a housing 520 that defines a longitudinal channel 522 and an internal chamber 524. The proximal end 514 of the elongate guide is at least partially threaded. A drive nut 530 is disposed in the internal chamber 524 configured for coaxial alignment with the proximal end 514 of the elongate guide 510. The drive nut 530 has internal threads 532 to selectively engage at least a portion of the threads disposed thereon the proximal end 514 of the elongate guide. The bore 534 of the drive nut 530 is sized such that it can be moved laterally into and out of engagement with the threads disposed thereon the proximal end of the elongate guide 510. It is configured to move from a first non-engaged position, where the housing can freely move along the longitudinal axis L_{G} of the elongate guide and, to a second engaged position, where the housing can move in a controlled manner along the longitudinal axis of the elongate guide by rotating the drive nut in either a clockwise or counterclockwise manner.

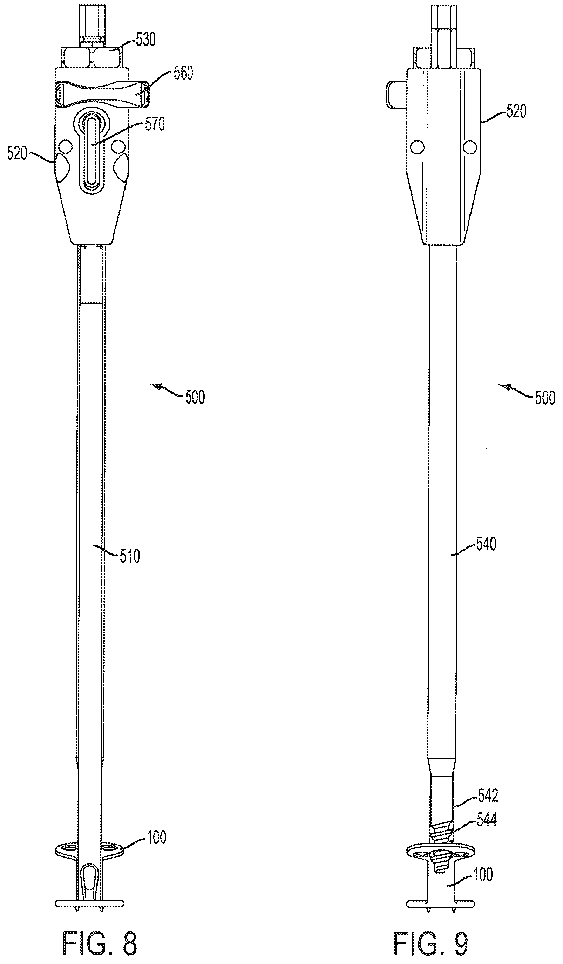

In one aspect, the lamina setting tool 500 comprises an elongate bone screw shaft 540 partially retained within the longitudinal channel 522 of the housing. The bone screw shaft 540 is configured for rotation within the longitudinal channel. As can be seen in Fig. 9, the distal end 542 of the bone screw shaft 540 comprises threads 544 for boring into a portion of a lamina of the desired cervical vertebra. In another aspect, the elongate guide 510 and the elongate bone screw shaft 540 are substantially parallel.

As mentioned, in one aspect, in the non-engaged position, the housing can freely move along the longitudinal axis of the elongate guide 510. As such, in one aspect, there is a stop mechanism 527 positioned thereon the elongate guide 510 substantially adjacent the proximal end 514 of the elongate guide to limit longitudinal movement of the housing toward the distal end 512 of the elongate guide.

In still another aspect, the lamina setting tool of can also have at least one bias element 550 positioned therein the internal chamber 524 of the housing substantially transverse to and external of the exterior portion 536 of the drive nut 530. The bias element can be, for example and not meant to be limiting, a spring. The bias element is designed, in this aspect, to bias the drive nut into engagement with at least a portion of the threads disposed thereon the proximal end of the elongate guide 510. Therefore, in the normal position, the drive nut would be in the engaged position.

To work with the bias element 550, in one aspect, the housing can define a lockslide recess 560 positioned substantially tangential to the exterior portion of the drive nut. Disposed therein the lockslide recess 560 is a lockslide 565 that has a ramped surface 567 configured to wedge against the exterior portion 536 of the drive nut and move it from the first non-engaged position to the second engaged position. The ramped surface can, for example, have a plurality of curved ramped surfaces to conform to a portion of the exterior portion of the drive nut.

In one exemplified aspect, the elongate bone screw shaft 540 is substantially restricted from longitudinal movement with respect to the channel. Thus, it moves longitudinally with the housing with respect to the elongate guide 510.

In another example, the drive nut can define a recess 538 in its exterior surface 536. The housing, in this aspect, can have a rocker arm aperture 526 defined therein that is longitudinally aligned with the recess 538 of the drive nut. As such, there can be a rocker arm 570 having a proximal end 572 hingedly affixed to an external portion 529 of the housing 520 and a distal end 574 positioned therethrough the rocker arm aperture 526. The distal end of the rocker arm can move into and out of engagement with the recess, as the recess rotates with the drive nut. In this manner, at least one time per revolution of the drive nut, the distal end of the rocker arm is disposed within the recess. This configuration gives the user a tactile sense of the relative movement of the housing, and thus the elongate bone screw shaft, to the elongate guide, as each rotation of the lock nut represents differential movement of the housing with respect to the elongate guide, depending upon the thread pitch of the threads on the proximal end of the elongate guide and the internal threads of the drive nut.

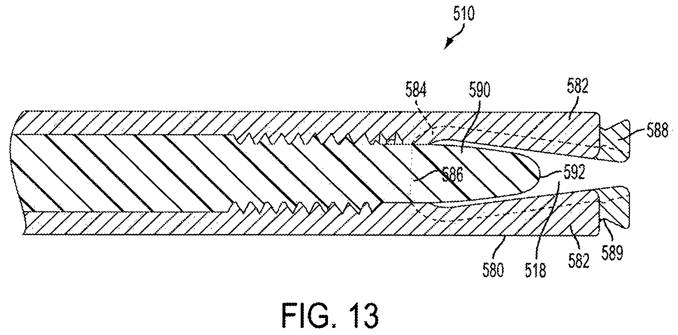

In one aspect, the elongate guide 510 can be substantially tubular, defining an internal longitudinal shaft cavity 518. In this aspect, the distal end of the elongate guide 512 comprises a tip 580 with an inwardly tapered horseshoe cross-sectional shape with two leg portions 582 and a seat portion 584 that defines a seat aperture 586, as illustrated in Fig. 13. The external portion of the tip 588 is configured to mate with an interior portion 175 of the tool bore 170 of the laminoplasty plate. In this aspect, an elongate rod 590 is disposed within the shaft cavity 518. The elongate rod has a distal end portion 592 that is configured to move longitudinally therethrough the seat aperture 586 to selectively splay the two leg portions 582 away from each other. As the outer diameter of the tip of the guide is increased, it causes the external portion 588 of the tip to frictionally engage the tool bore 170. In one aspect, the external portion of the tip also has a circumferential ridge 589 within which the interior portion of the tool bore can mate. As illustrated, in one aspect, the distal end portion 592 of the elongate rod is threaded and configured to engage the seat aperture such that rotation of the elongate red 590 causes the distal end portion of the elongate rod to protrude into and retract from engagement with the two leg portions, spaying the leg portions in one direction and allowing them to retract to the normal position in the other direction.

As can be seen in Fig. 7, the lamina setting tool 500 in this aspect can comprise graduated markings 594 on the external portion of the elongate guide 510, as well as on the housing to display visually the boring depth of the distal end 542 of the elongate bone screw shaft 540 into the portion of a lamina of a cervical vertebra, and to assist in determining the relative longitudinal location of the elongate bone screw shaft with respect to the elongate guide 510.

Also presented herein are methods of treating cervical stenosis in a patient by relieving spinal cord compression. In one aspect, at least a portion of the desired cervical vertebra, which defines a spinal canal 240 having a pre-operative cross-sectional area, is exposed. To do so, a posterior incision in the patient over an area of cervical stenosis of the patient is made to expose the posterior side of the desired cervical vertebra. A small pathway, ranging from about 14 to 18 mm, can be dilated through the soft tissue to reach the desired cervical vertebra 200 so that muscle and tissue damage is kept to a minimum. The spine may be exposed more extensively in the traditional open approach.

The first lamina portion of the desired cervical vertebra can be separated. In one example the step of separating the first lamina portion 210 of the desired cervical vertebra comprises making a first sagittal division 225 from the exterior of the desired cervical vertebra to the spinal canal on a first side of the midline of the vertebra and making a second sagittal division 227 from the exterior of the desired cervical vertebra to the spinal canal on a second side of midline 260. Thus, the lamina portion 210 of the desired cervical vertebra 200 and the spinous process 270 will no longer be attached at any point to the remainder of the desired cervical vertebra. The first and second sagittal divisions are made at the junction between the lamina portion and the lateral mass portion.

The first lamina portion can be controllably elevated to a relief position in which the spinal canal of the desired cervical vertebra has a relief cross-sectional area that is greater than the pre-operative cross-sectional area, wherein the first lamina portion is subsequently secured in an elevated position. After providing at least one laminoplasty plate, the step of controllably raising the first lamina portion 210 to a relief position can first comprise attaching at least a portion of the distal end portion of a first laminoplasty plate to a portion of the first lateral mass portion of the desired cervical vertebra adjacent the first sagittal division 225 and attaching at least a portion of the proximal end portion of the first laminoplasty plate to a first lamina portion adjacent the first sagittal division. A pre-determined length of the medial portion 130 of the laminoplasty plate 100 can correspond to the amount of separation needed between lamina portion and the desired cervical vertebra. The step of controllably raising the second lamina portion 215 to the relief position can then comprise attaching at least a portion of the distal end portion of a second laminoplasty plate to a second lateral mass portion of the desired cervical vertebra adjacent the second sagittal division 227 and attaching at least a portion of the proximal end portion 110 of the second laminoplasty plate to a second lamina portion adjacent the second sagittal division. In one example
the laminoplasty plates can be attached to the desired lateral mass portion and lamina portion with screws. In this example, the screws can be conventional self-tapping bone screws. It is also contemplated that conventional non-self-tapping bone screws can be used. The step of attaching the distal end portion 105 of the laminoplasty plates to the desired cervical vertebra comprises attaching the distal end portion of the laminoplasty plates to the respective lateral mass of the cervical vertebra. It is also contemplated that the steps of the method described herein can be completed on the first side of the midline and the second side of the midline simultaneously, sequentially, or in an alternating fashion.

The lamina setting tool 300 can be provided to assist with the step of controllably raising and securing the lamina portion in the relief position. The guide is configured to detachably mount the mountable portion 160 of the laminoplasty plate 100. In one example, the guide can comprise a hollow shank portion configured to interlock with the raised cone portion of the distal end portion of the laminoplasty plate. In another example, the guide can comprise a mounting edge adapted to be secured within the cavity of the distal end portion. It is contemplated that the present invention can encompass alternative means for mounting the guide to the mountable portion of the laminoplasty plate.

As noted above, the laminoplasty plate comprises a proximal end portion that can define a tool screw aperture 150 configured for operative receipt of the lamina setting tool. The rotatable threaded shaft 310 of the lamina setting tool is configured for insertion into the tool screw aperture. The distance between the rotatable threaded shaft and the body portion 332 of the guide 330 can be equal to the distance between the tool screw aperture and the mountable portion of the distal end portion.

The lamina portion 210 of the desired cervical vertebra can be separated by making a first sagittal division from the exterior of the desired cervical vertebra 200 to the spinal canal on a first side of the midline of the spinous process 270 and making a second sagittal division from the exterior of the desired cervical vertebra to the spinal canal on a second side of midline. The second sagittal division may be a partial thickness division, leaving a portion of the second portion of the lamina partially intact. Only the first laminoplasty plate would be used and the second sagittal division 227 would substantially hinge, permitting movement of the first lamina portion 210.

Following the separation of the lamina portion, the distal end portion of a first laminoplasty plate can be secured to the portion of the desired cervical vertebra adjacent the first sagittal division. The guide detachably mounts the mountable portion 160 of the first laminoplasty plate. The surgeon can line up the rotatable threaded shaft with the tool screw aperture on the proximal end portion of the first laminoplasty plate. The surgeon rotates the rotatable threaded shaft through the tool screw aperture and through the lamina portion until the stop contacts the first support arm of the guide at an interference point. At this point, further rotation of the rotatable threaded shaft controls the elevation of the first lamina portion. Accordingly, during this process, the stop must be securely attached to the rotatable threaded shaft such that further insertion of the rotatable threaded shaft into the lamina portion is prevented. The surgeon rotates the rotatable threaded shaft until the top surface of the lamina portion is substantially flush with the bottom surface 120 of the proximal end portion of the first laminoplasty plate. The proximal end portion of the first laminoplasty plate is then secured to the lamina portion, wherein the lamina portion is fixed in the relief position. It is contemplated that this procedure can then be completed on the second side of midline 260 about the second sagittal division through the use of a second laminoplasty plate, if a complete division of the lamina is performed bilaterally.

The rotatable threaded shaft can have an outer diameter and an inner diameter, with the outer diameter being smaller than the pre-determined diameter of the bore configured for operative receipt of the lamina setting tool. The rotatable threaded shaft can have a thread-pitch equal to the distance between the threads along the shaft. The thread pitch can be determined once the outer and inner diameters of the rotatable threaded shaft are known, with the range of outer diameters preferably falling between 0 and 16 mm, more preferably between 1 and 4 mm, and with the range of inner diameters preferably falling between 0 and 14 mm, more preferably between 0.5 and 3.5 mm. Under these constraints, the thread pitch will preferably fall between 0 and 4 mm, more preferably between 0.25 and 2 mm. As one will appreciate, the thread-pitch can be used to determine the amount of rotation of the rotatable threaded shaft 310 required to accomplish a given lift distance. Accordingly, the gauge of the lamina setting tool 300 can be calibrated based on the thread-pitch of the rotatable threaded shaft.

The step of attaching the rotatable threaded shaft to the lamina portion comprise can drilling a hole in the first and second sides of midline adjacent the first and second sagittal divisions and screwing the rotatable threaded shaft into the holes defined by the first and second sides of midline. As one skilled in the art will appreciate, the diameter of the drilled holes must be large enough to allow for the insertion of the rotatable threaded shaft.

The lamina setting tool 500 can be provided to assist with the step of controllably raising and securing the lamina portion in the relief position. The laminoplasty plate can be secured to the tip of the elongate guide 510 by placing the tip into the tool bore 170 of the laminoplasty plate and splaying the legs of the tip into frictional engagement with the interior portion 175 of the tool bore 170. Using the guide, the laminoplasty plate can be positioned onto a lateral mass portion of the desired cervical vertebra, adjacent a partial sagittal division which has already been performed substantially at the junction between the lateral mass and the lamina. To assist in holding the laminoplasty plate in position, the laminoplasty plate can comprise one or more spikes 178 protruding from the bottom surface of the distal end portion of the plate. The laminoplasty plate can then be secured to the lateral mass using screws.

In another aspect, the housing can be placed onto the elongate guide by placing the lock nut in the non-engaged position and sliding over the proximal end of the elongate guide, ensuring that the elongate bone screw shaft is coaxial with the tool screw aperture 150. Keeping the lock nut in the non-engaged position, the elongate bone screw shaft can then be driven into the lamina to the desired depth. By knowing the distance between the first and second planes of the laminoplasty plate, the markings on the housing and the external portion of the elongate guide 510, the surgeon can determine the depth of the distal end of the elongate bone screw shaft.

In this aspect, the lamina can be completely separated at the sagittal division from the lateral mass. At this point, the drive nut can be placed in the engaged position. In one aspect, this can be completed by repositioning the lockslide to overcome the force of the bias element. The lamina can now be controllably raised by rotating the drive nut and raising the elongate bone screw shaft with respect to the elongate guide.

Once the lamina is raised to the relief position, as indicated by the markings 528 on the housing and markings 594 on the external portion of the elongate guide, it can be secured into position by placing screws into the screw bores on the proximal end portion of the laminoplasty plate. Once secured, the elongate bone screw shaft can be removed, as well as the elongate guide. As one skilled in the art can appreciate, these steps can be varied with respect to sequence by the surgeon, as need. The method can also be performed bilaterally or using the aforementioned open door procedure.

A graft (not shown) can be placed proximate at least a portion of the distal and proximal end portions of the plurality of laminoplasty plates to allow fusion of the lamina portion in the relief position. In this example, the graft can be configured to surround at least a portion of the medial portion of the laminoplasty plates. In a specific example, the medial portion of the laminoplasty plate can be of reduced cross-sectional area relative to the distal and proximal end portions, and the graft can be substantially U-shaped to substantially surround the medial portion of the laminoplasty plate. Further, the graft can be composed of autologous bone, allograft bone, synthetic bone substitute, and osteoinductive agent.

Although several aspects of the invention have been disclosed in the foregoing specification, it is understood by those skilled in the art that many modifications and other aspects of the invention will come to mind to which the invention pertains, having the benefit of the teaching presented in the foregoing description and associated drawings. It is thus understood that the invention is not limited to the specific aspects disclosed hereinabove, and that many modifications and other aspects are intended to be included within the scope of the appended claims. Moreover, although specific terms are employed herein, as well as in the claims that follow, they are used only in a generic and descriptive sense, and not for the purposes of limiting the described invention.

## Claims

1. A system for performing a laminoplasty for treating cervical stenosis in a patient, the system comprising:
a laminoplasty plate (100) having a medial portion (130) and comprising:
a proximal end portion (110) having a bottom surface (120) defined in a first plane; and
a distal end portion (105) having a bottom surface (120) defined in a second plane,
wherein, at the medial portion (130), the first plane and second plane are spaced from each other; and
a means for fastening the distal end portion (105) of the laminoplasty plate to a lateral mass portion of a desired cervical vertebra of the patient;
a lamina setting tool (500) comprising:
a means for fastening the proximal end portion (110) of the laminoplasty plate (100) to the lamina portion in the relief position;
**characterized by** the lamina setting tool (500) further comprising:
an elongate guide (510) with a longitudinal axis, and a distal end (512) and a proximal end (514), wherein the distal end (512) is configured to engage portions of the laminoplasty plate (100) and for releasably engaging the distal end (512) portion of the laminoplasty plate (100), and wherein the proximal end (514) is at least partially threaded;
a housing (520) defining a longitudinal channel (522) and an internal chamber (524);
a means for controllably raising a lamina portion separated from the lateral mass portion to a relief position, wherein the means comprises a drive nut (530) disposed in the internal chamber (524) having internal threads configured for coaxial alignment with the proximal end (514) of the elongate guide (510), wherein the drive nut (530) is configured to selectively engage at least a portion of the threads disposed thereon the proximal end (514) of the elongate guide (510), wherein, in a first non-engaged position, the housing (520) can freely move along the longitudinal axis of the proximal end (514) of the elongate guide (510) and, in a second engaged position, the housing (520) can move in a controlled manner along the longitudinal axis of the proximal end (514) of the elongate guide (510) by rotating the drive nut (530) in either a clockwise or counterclockwise manner;
and an elongate bone screw shaft (540) partially retained within the longitudinal channel (522) and configured for rotation within the longitudinal channel (522), wherein a distal end (542) of the bone screw shaft (540) comprises threads (544) for boring into a portion of a lamina of a cervical vertebra.

2. The system of claim 1, wherein at least a portion of the first plane is substantially parallel to at least a portion of the second plane, or
wherein at least a portion of the first plane is at an acute angle to at least a portion of the second plane, or
wherein the medial portion (130) of the laminoplasty plate (100) has a reduced cross-sectional area relative to the cross-sectional areas of the distal (105) and proximal (110) end portions.

3. The system of claim 1, wherein the lamina setting tool further comprises a stop mechanism (527) positioned thereon the elongate guide (510) substantially adjacent the proximal end (514) of the elongate guide (510) to limit longitudinal movement of the housing (522) toward the distal end (512) of the elongate guide (510).

4. The system of claim 1, wherein the elongate guide (510) and the elongate bone screw shaft (540) are substantially parallel.

5. The system of claim 1, wherein the lamina setting tool (500) further comprises at least one bias element (550) positioned therein the internal chamber (524) of the housing (520) substantially transverse to and external of an exterior portion (536) of the drive nut (530), wherein the at least one bias element (550) is configured to bias the drive nut (530) into engagement with at least a portion of the threads disposed thereon the proximal end (514) of the elongate guide (510).

6. The system of claim 5, wherein the housing (520) defines a lockslide recess (560) positioned substantially tangential to the exterior portion (536) of the drive nut (530) and comprises a lockslide (565) disposed therein the lockslide recess (560), wherein the lockslide (565) has a ramped surface (567) configured to wedge against the exterior portion (536) of the drive nut (530) and move it from the first non-engaged position to the second engaged position.

7. The system of claim 1, wherein the elongate bone screw shaft (540) is substantially restricted from longitudinal movement with respect to the channel (522).

8. The system of claim 1, wherein the drive nut (530) defines a recess (538) in an exterior surface (536) of the drive nut.

9. The system of claim 8, wherein the lamina setting tool (500) further comprises a rocker arm (570) having a proximal end (572) hingedly affixed to an external portion (529) of the housing (520) and a distal end (574) positioned therethrough a rocker arm aperture (526) defined in the housing (520), wherein the distal end (574) of the rocker arm (570) is longitudinally aligned with the recess (538) in the exterior surface (536) of the drive nut (530) and is biased against the recess (538) such that, at least one time per revolution of the drive nut (530), the distal end (574) of the rocker arm (570) is disposed within the recess (538).

10. The system of claim 1, wherein the elongate guide (510) is substantially tubular, defining an internal longitudinal shaft cavity (518), and wherein the distal end (512) of the elongate guide (510) comprises a tip (580) with an inwardly tapered horseshoe cross-sectional shape with two leg portions (582) and a seat portion (584) that defines a seat aperture (586).

11. The system of claim 10, wherein the lamina setting tool (500) further comprises an elongate rod (590) disposed within the shaft cavity (518), the elongate rod (590) having a distal end portion (592) that is configured to move longitudinally therethrough the aperture (586) to selectively splay the two leg portions (582) away from each other.

12. The system of claim 11, wherein the distal end portion (592) of the elongate rod (590) is threaded and configured to engage the seat aperture (586) such that rotation of the elongate rod (590) causes the distal end portion (592) of the elongate rod (590) to protrude into and retract from engagement with the two leg portions (582).

13. The system of claim 1, wherein the lamina setting tool (500) further comprises a means for determining the boring depth of a distal end (542) of the elongate bone screw shaft (540) into the portion of a lamina of a cervical vertebra.

14. The system of claim 13, wherein the lamina setting tool (500) further comprises a means for determining the relative longitudinal location of the elongate bone screw shaft (540) with respect to the elongate guide (510).

## Patentansprüche

1. System zum Durchführen einer Laminoplastik zur Behandlung von Zervixstenose in einem Patienten, das System aufweisend:
eine Laminoplastik-Platte (100) mit einen mittleren Abschnitt (130), und die
einen proximalen Endabschnitt (110) mit einer unteren Oberfläche (120), die in einer ersten Ebene definiert ist; und
einen distalen Endabschnitt (105) mit einer unteren Oberfläche (120), die in einer zweiten Ebene definiert ist,
wobei beim mittleren Abschnitt (130) die erste Ebene und die zweite Ebene voneinander beabstandet sind; und
ein Mittel zum Befestigen des distalen Endabschnitts (105) der Laminoplastik-Platte an einem seitlichen Masseteil eines gewünschten Halswirbels des Patienten aufweist;
ein Lamina-Einstellwerkzeug (500), das
ein Mittel zum Befestigen des proximalen Endabschnitts (110) der Laminoplastik-Platte (100) an dem Lamina-Abschnitt in der Entlastungsstellung aufweist;
**dadurch gekennzeichnet, dass** das Lamina-Einstellwerkzeug (500) ferner
eine längliche Führung (510) mit einer Längsachse, und einem distalen Ende (512) und einem proximalen Ende (514), wobei das distale Ende (512) so ausgebildet ist, dass es mit Abschnitten der Laminoplastik-Platte (100) im Eingriff steht, und dass es im lösbaren Eingriff mit dem distalen EndAbschnitt (512) der Laminoplastik-Platte (100) steht, und wobei das proximale Ende (514) zumindest teilweise mit Gewinde versehen ist;
ein Gehäuse (520), das einen Längskanal (522) und eine innere Kammer (524) definiert;
ein Mittel zum steuerbaren Anheben eines Lamina-Abschnitts, der von dem seitlichen Masseteil getrennt ist, in eine Entlastungsstellung, wobei das Mittel eine Gegenmutter (530) aufweist, die in der inneren Kammer (524) angeordnet ist, die ein Innengewinde hat, das für eine koaxiale Ausrichtung mit dem proximalen Ende (514) der länglichen Führung (510) ausgebildet ist, wobei die Gegenmutter (530) ausgebildet ist, um selektiv zumindest in einen Abschnitt der auf dem proximale Ende (514) der länglichen Führung (510) angeordneten Gewinde einzugreifen, wobei sich das Gehäuse (520), in einer ersten nicht-eingreifenden Stellung, entlang der Längsachse des proximalen Endes (514) der länglichen Führung (510) frei bewegen kann, und sich das Gehäuse (520), in einer zweiten eingreifenden Stellung, in einer kontrollierten Weise entlang der Längsachse des proximalen Endes (514) der länglichen Führung (510) durch Drehung der Gegenmutter (530) entweder im Uhrzeigersinn oder gegen den Uhrzeigersinn bewegen kann;
und einen länglichen Knochenschraubenschaft (540) aufweist, der teilweise innerhalb des Längskanals einbehalten (522) und zur Drehung innerhalb des Längskanals (522) ausgebildet ist, wobei ein distales Ende (542) des Knochenschraubenschafts (540) Gewinde (544) zum Bohren in einen Abschnitt einer Lamina eines Halswirbels aufweist.

2. System nach Anspruch 1, wobei zumindest ein Abschnitt der ersten Ebene im Wesentlichen parallel ist zu zumindest einem Abschnitt der zweiten Ebene, oder
wobei zumindest ein Abschnitt der ersten Ebene in einem spitzen Winkel zu zumindest einem Abschnitt der zweiten Ebene ist, oder
wobei der mittlere Abschnitt (130) der Laminoplastik Platte (100) eine reduzierte Querschnittsfläche relativ zu den Querschnittsflächen der distalen (105) und proximalen (110) Endabschnitten hat.

3. System nach Anspruch 1, wobei das Lamina-Einstellwerkzeug ferner einen Stoppmechanismus (527) aufweist, der auf der länglichen Führung (510) im Wesentlichen angrenzend an das proximale Ende (514) der länglichen Führung (510) positioniert ist, um Längsbewegung des Gehäuses (522) zum distalen Ende (512) des länglichen Führung (510) zu begrenzen.

4. System nach Anspruch 1, wobei die längliche Führung (510) und der längliche Knochenschraubenschaft (540) im Wesentlichen parallel sind.

5. System nach Anspruch 1, wobei das Lamina-Einstellwerkzeug (500) ferner mindestens ein Vorspannelement (550) aufweist, das in der inneren Kammer (524) des Gehäuses (520) im Wesentlichen quer zu und außerhalb eines äußeren Abschnitts (536) der Gegenmutter (530) positioniert ist, wobei das mindestens eine Vorspannelement (550) so ausgebildet ist, dass die Gegenmutter (530) zum Eingriff mit mindestens einem Abschnitt der auf dem proximalen Ende (514) der länglichen Führung (510) angeordneten Gewinde vorgespannt ist.

6. System nach Anspruch 5, wobei das Gehäuse (520) eine Sicherungsschieber-Aussparung (560) definiert, die im Wesentlichen tangential an dem Außenabschnitt (536) der Gegenmutter (530) angeordnet ist, und einen Sicherungsschieber (565) aufweist, der in der Sicherungsschieber-Aussparung (560) angeordnet ist, wobei der Sicherungsschieber (565) eine abgeschrägte Oberfläche (567) hat, die ausgebildet ist, um sich mit dem äußeren Abschnitt (536) der Gegenmutter (530) zu verkeilen und sie von der ersten nicht-eingreifenden Stellung in die zweite eingreifende Stellung zu bewegen.

7. System nach Anspruch 1, wobei die Längsbewegung des länglichen Knochenschraubenschafts (540) in Bezug auf den Kanal (522) im Wesentlichen beschränkt ist.

8. System nach Anspruch 1, wobei die Gegenmutter (530) eine Aussparung (538) in einer äußeren Oberfläche (536) der Gegenmutter definiert.

9. System nach Anspruch 8, wobei das Lamina-Einstellwerkzeug (500) weiter einen Kipphebel (570) mit einem proximalen Ende (572) aufweist, der schwenkbar an einem äußeren Abschnitt (529) des Gehäuses (520) befestigt ist, und einem distalen Ende (574), das durch eine in dem Gehäuse (520) definierte Kipphebel-Öffnung (526) hindurch positioniert ist, wobei das distale Ende (574) des Kipphebels (570) in Längsrichtung an der Aussparung (538) in der äußeren Oberfläche (536) der Gegenmutter (530) ausgerichtet ist, und so gegen die Aussparung (538) vorgespannt ist, dass, mindestens einmal pro Umdrehung der Gegenmutter (530), das distale Ende (574) des Kipphebels (570) innerhalb der Aussparung (538) angeordnet ist.

10. System nach Anspruch 1, wobei die längliche Führung (510) im Wesentlichen rohrförmig ist, einen inneren Längsschafthohlraum (518) definiert, und wobei das distale Ende (512) der länglichen Führung (510) eine Spitze (580) mit einer nach innen verjüngten Hufeisen-Querschnittsform mit zwei Schenkelabschnitten (582) und einem Basisabschnitt (584), der eine Basisöffnung (586) definiert, aufweist.

11. System nach Anspruch 10, wobei das Lamina-Einstellwerkzeug (500) ferner eine längliche Stange (590) innerhalb des Schafthohlraums (518) aufweist, wobei die längliche Stange (590) einen distalen Endabschnitt (592) aufweist, der so ausgebildet ist, dass er sich in Längsrichtung durch die Öffnung (586) bewegen kann, um die beiden Schenkelabschnitte (582) selektiv voneinander weg zu spreizen.

12. System nach Anspruch 11, wobei der distale Endabschnitt (592) der länglichen Stange (590) mit Gewinde versehen ist und ausgebildet ist, um so mit der Basisöffnung (586) in Eingriff zu stehen, dass Drehung der länglichen Stange (590) bewirkt, dass der distale Endabschnitt (592) der länglichen Stange (590) in die beiden Schenkelabschnitte (582) hineinragt und nicht mehr mit diesen in Eingriff steht.

13. System nach Anspruch 1, wobei das Lamina-Einstellwerkzeug (500) ferner ein Mittel zur Bestimmung der Bohrtiefe eines distalen Endes (542) des länglichen Knochenschraubenschafts (540) in den Abschnitt einer Lamina eines Halswirbels hinein aufweist.

14. System nach Anspruch 13, wobei das Lamina-Einstellwerkzeug (500) ferner ein Mittel zur Bestimmung der relativen Längsposition des länglichen Knochenschraubenschafts (540) in Bezug auf die längliche Führung (510) aufweist.

## Revendications

1. Système destiné à réaliser une laminoplastie pour traiter une sténose cervicale chez un patient, le système comprenant :
une plaque de laminoplastie (100) ayant une partie médiale (130) et comprenant :
une partie d'extrémité proximale (110) ayant une surface inférieure (120) définie dans un premier plan ; et
une partie d'extrémité distale (105) ayant une surface inférieure (120) définie dans un deuxième plan,
dans lequel, au niveau de la partie médiale (130), le premier plan et le deuxième plan sont écartés l'un de l'autre ; et
un moyen destiné à fixer la partie d'extrémité distale (105) de la plaque de laminoplastie à une partie de masse latérale d'une vertèbre cervicale souhaitée du patient ;
un instrument de mise en place de lame (500) comprenant :
un moyen destiné à fixer la partie d'extrémité proximale (110) de la plaque de laminoplastie (100) à la partie de lame dans la position de repos ;
**caractérisé en ce que** l'instrument de mise en place de lame (500) comprend en outre :
un guide allongé (510) avec un axe longitudinal, et une extrémité distale (512) et une extrémité proximale (514), dans lequel l'extrémité distale (512) est configurée pour s'engager avec des parties de la plaque de laminoplastie (100) et pour s'engager de façon libérable avec la partie d'extrémité distale (512) de la plaque de laminoplastie (100), et dans lequel l'extrémité proximale (514) est au moins partiellement filetée ;
un logement (520) définissant un canal longitudinal (522) et une chambre interne (524) ;
un moyen destiné à soulever de manière contrôlée une partie de lame séparée de la partie de masse latérale à une position de repos, dans lequel le moyen comprend un écrou d'entraînement (530) disposé dans la chambre interne (524) ayant des filetages internes configurés pour un alignement coaxial avec l'extrémité proximale (514) du guide allongé (510), dans lequel l'écrou d'entraînement (530) est configuré pour s'engager sélectivement avec au moins une partie des filetages disposés sur l'extrémité proximale (514) du guide allongé (510), dans lequel, dans une première position non engagée, le logement (520) peut librement se déplacer le long de l'axe longitudinal de l'extrémité proximale (514) du guide allongé (510) et, dans une deuxième position engagée, le logement (520) peut se déplacer d'une manière contrôlée le long de l'axe longitudinal de l'extrémité proximale (514) du guide allongé (510) en faisant tourner l'écrou d'entraînement (530) soit dans le sens des aiguilles d'une montre soit dans le sens inverse des aiguilles d'une montre ;
et un axe de vis à os allongé (540) partiellement retenu dans le canal longitudinal (522) et configuré pour une rotation avec le canal longitudinal (522), dans lequel une extrémité distale (542) de l'axe de vis à os (540) comprend des filetages (544) pour percer dans une partie d'une lame d'une vertèbre cervicale.

2. Système selon la revendication 1, dans lequel au moins une partie du premier plan est substantiellement parallèle à au moins une partie du deuxième plan, ou
dans lequel au moins une partie du premier plan se trouve à un angle aigu par rapport à au moins une partie du deuxième plan, ou
dans lequel la partie médiale (130) de la plaque de laminoplastie (100) a une surface en section transversale réduite par rapport aux surfaces en section transversale des parties d'extrémité distale (105) et proximale (110).

3. Système selon la revendication 1, dans lequel l'instrument de mise en place de lame comprend en outre un mécanisme d'arrêt (527) positionné sur le guide allongé (510) à une position substantiellement adjacente à l'extrémité proximale (514) du guide allongé (510) pour limiter un mouvement longitudinal du logement (522) en direction de l'extrémité distale (512) de guide allongé (510).

4. Système selon la revendication 1, dans lequel le guide allongé (510) et l'axe de vis à os (540) sont substantiellement parallèles.

5. Système selon la revendication 1, dans lequel l'instrument de mise en place de lame (500) comprend en outre au moins un élément de sollicitation (550) positionné dans la chambre interne (524) du logement (520) de manière substantiellement transversale par rapport à l'extérieur d'une partie extérieure (536) de l'écrou d'entraînement (530), dans lequel le au moins un élément de sollicitation (550) est configuré pour solliciter l'écrou d'entraînement (530) pour l'amener en engagement avec au moins une partie des filetages disposés sur l'extrémité proximale (514) du guide allongé (510).

6. Système selon la revendication 5, dans lequel le logement (520) définit un évidement de glissière de verrouillage (560) positionné de manière substantiellement tangentielle par rapport à la partie extérieure (536) de l'écrou d'entraînement (530) et comprend une glissière de verrouillage (565) disposée dans l'évidement de glissière de verrouillage (560), dans lequel la glissière de verrouillage (565) a une surface inclinée (567) configurée pour se caler contre la partie extérieure (536) de l'écrou d'entraînement (530) et pour le déplacer de la première position non engagée vers la deuxième position engagée.

7. Système selon la revendication 1, dans lequel l'axe de vis à os allongé (540) est substantiellement limité en ce qui concerne un mouvement longitudinal par rapport au canal (522).

8. Système selon la revendication 1, dans lequel l'écrou d'entraînement (530) définit un évidement (538) dans une surface extérieure (536) de l'écrou d'entraînement.

9. Système selon la revendication 8, dans lequel l'instrument de mise en place de lame (500) comprend en outre un bras oscillant (570) ayant une extrémité proximale (572) fixée de manière articulée sur une partie externe (529) du logement (520) et une extrémité distale (574) positionnée à travers une ouverture de bras oscillant (526) définie dans le logement (520), dans lequel l'extrémité distale (574) du bras oscillant (570) est longitudinalement alignée avec l'évidement (538) dans la surface extérieure (536) de l'écrou d'entraînement (530) et est sollicitée contre l'évidement (538) de sorte que, au moins une fois par révolution de l'écrou d'entraînement (530), l'extrémité distale (574) du bras oscillant (570) est disposée dans l'évidement (538).

10. Système selon la revendication 1, dans lequel le guide allongé (510) est substantiellement tubulaire, en définissant une cavité d'axe longitudinale interne (518) et dans lequel l'extrémité distale (512) du guide allongé (510) comprend une pointe (580) avec une forme en section transversale de fer à cheval incliné vers l'intérieur avec deux parties de jambes (582) et une partie de siège (584) qui définit une ouverture de siège (586).

11. Système selon la revendication 10, dans lequel l'instrument de mise en place de lame (500) comprend en outre une tige allongée (590) disposée dans la cavité d'axe (518), la tige allongée (590) ayant une partie d'extrémité distale (592) qui est configurée pour se déplacer longitudinalement à travers l'ouverture (586) pour sélectivement écarter les deux parties de jambes (582) l'une par rapport à l'autre.

12. Système selon la revendication 11, dans lequel la partie d'extrémité distale (592) de la tige allongée (590) est filetée et configurée pour s'engager avec l'ouverture de siège (586) de sorte qu'une rotation de la tige allongée (590) amène la partie d'extrémité distale (592) de la tige allongée (590) à dépasser et à se rétracter par rapport à un engagement avec les deux parties de jambes (582).

13. Système selon la revendication 1, dans lequel l'instrument de mise en place de lame (500) comprend en outre un moyen destiné à déterminer la profondeur de perçage d'une extrémité distale (542) de l'axe de vis à os allongé (540) dans la partie d'une lame d'une vertèbre cervicale.

14. Système selon la revendication 13, dans lequel l'instrument de mise en place de lame (500) comprend en outre un moyen destiné à déterminer la position longitudinale relative de l'axe de vis à os allongé (540) par rapport au guide allongé (510).
